# FASCICULE DE BREVET EUROPEEN

(11) **EP 0 665 057 B1**
(45) Date de publication et mention de la délivrance du brevet: **15.12.1999**
(21) Numéro de dépôt: 95400041.0
(22) Date de dépôt: 10.01.1995
(51) Int. Cl.: B01J 31/22, C07C 6/04

(54) **Composition catalytique et procédé pour la disproportion des oléfines**
Katalytische Zusammensetzung und Verfahren zur Disproportionierung von Olefinen
Catalytic composition and process for the disproportionation of olefins

(30) Priorité: 26.01.1994 FR 9400823
(43) Date de publication de la demande: 02.08.1995
(73) Titulaire: INSTITUT FRANCAIS DU PETROLE, 92502 Rueil-Malmaison (FR)
(72) Inventeur: Chauvin, Yves, F-78230 Le Pecq (FR); Di Marco van Tiggelen, Françoise, F-92000 Nanterre (FR)

(56) Documents cités:
- EP-A- 0 129 474
- EP-A- 0 283 400
- EP-A- 0 448 445
- US-A- 3 855 340

## Description

La présente invention concerne une composition catalytique et un procédé utilisant cette composition pour la réaction de disproportion, dite encore métathèse, des oléfines. Cette composition résulte de la dissolution d'un composé du tungstène ou de molybdène dans le mélange liquide, a caractère ionique, d'halogénure d'ammonium quaternaire ou/et d'halogénure de phosphonium quaternaire, d'halogénure d'aluminium et d'un composé d'alcoyle aluminium.

La réaction de disproportion des oléfines est couramment catalysée par des catalyseurs typiquement hétérogènes, tels que les oxydes de molybdène, de tungstène ou de rhénium déposés sur la silice ou l'alumine, ou par des catalyseurs organométalliques, dits homogènes, qui résultent de la combinaison d'halogénures ou d'halogénures substitués du tungstène et du molybdène avec des composés organométalliques de la série principale et plus particulièrement les composés organiques de l'aluminium. Ainsi différents systèmes organométalliques ont été décrits par H.T. Dodd et K.J. Rutt dans Journal of Molecular Catalysis, 15, 1982, pp103-110.

Plus récemment il a été montré par J.M. Basset *et al*. dans le brevet U.S. 4 550 216 qu'en milieu chlorobenzène le produit de l'interaction d'un composé halogéné de tungstène comportant deux groupements phénoxo eux-mêmes substitués par des groupements électronégatifs en positions ortho, avec des organométalliques de la série principale étaient particulièrement actifs pour catalyser la métathèse des oléfines. Ce système souffre cependant de ne pas utiliser toutes les possibilités du catalyseur avant sa destruction.

Dans le brevet U.S. 5.104.840 est décrite une composition liquide à caractère ionique résultant de la mise en contact d'halogénures d'ammonium quaternaires et/ou d'halogénures de phosphonium quaternaires avec des dihalogénures d'alcoyle aluminium et éventuellement en outre un trihalogénure d'aluminium. Ce même brevet décrit l'utilisation de ces milieux comme solvants de complexes de métaux de transition, notamment des complexes du nickel ne contenant pas de liaison nickel-carbone, qui sont transformés en catalyseurs d'oligomérisation des oléfines. L'utilisation de tels milieux non miscibles avec les hydrocarbures aliphatiques, en particulier avec les produits issus de la transformation des oléfines permet une meilleure séparation des produits donc améliore l'utilisation des catalyseurs homogènes Dans la suite ces milieux seront appelés "sels fondus" parce que liquides à température modérée.

Il a maintenant été trouvé que les halogénures du tungstène ou du molybdène substitués par un ou deux groupements phénoxo contenant au moins deux substituants hydrocarbonés en positions ortho,ortho', dissous dans des "sels fondus" sont des catalyseurs efficaces pour la disproportion des oléfines. Cette formulation s'est révélée particulièrement intéressante parce que les complexes du tungstène ou du molybdène qui y sont dissous ne sont pas solubles dans les oléfines, présentent une activité catalytique importante et peuvent être utilisés pendant toute leur période d'activité.

Plus précisément un objet de l'invention est une composition catalytique comprenant au moins un composé du tungstène ou de molybdène, et plus particulièrement un halogénure du tungstène ou de molybdène substitué par un ou deux groupements phénoxo eux-mêmes substitués en ortho, ortho' par deux groupements hydrocarbonés, dissous au moins en partie dans un milieu non aqueux à caractère ionique résultant de la mise en contact d' un halogénure d'ammonium quaternaire et/ou au moins un halogénure de phosphonium quaternaire (noté produit A), avec au moins un halogénure d'aluminium (noté produit B) et avec au moins un composé organométallique de l'aluminium (noté produit C).

Un autre objet de l'invention est un procédé pour la disproportion ou la co-disproportion d'au moins une oléfine, procédé dans lequel l'oléfine est mise au contact d'au moins un composé du tungstène ou du molybdène ledit composé étant dissous au moins en partie dans le milieu non-aqueux à caractère ionique, ledit milieu résulte de la mise en contact d'au moins un halogénure d'aluminium avec au moins un halogénure d'ammonium quaternaire et/ou de phosphonium quaternaire et avec un dérivé organométallique de l'aluminium.

Le milieu de type "sels fondus" est donc constitué:
a) d'halogénures, plus particulièrement chlorures et/ou bromures, d'ammonium quaternaires et/ou phosphonium quaternaires (noté produit A);
b) d'halogénure d'aluminium, et plus particulièrement le chlorure ou le bromure d'aluminium (noté produit B);
d) d'un dérivé organique de l'aluminium (noté produit C).

Les halogénures d'ammonium quaternaires et les halogénures de phosphonium quaternaires utilisables dans le cadre de l'invention répondent de préférence aux formules générales NR^{I}R²R³R⁴X et PR^{I}R²R³R⁴X, où X représente C_{I} ou Br, R^{I}, R², R³ et R⁴, identiques ou différents, représentant chacun l'hydrogène, un groupement alkyle, aliphatique (saturé ou insaturé) ou aromatique, comprenant 1 a 12 atomes de carbone. Les halogénures d'ammonium et/ou de phosphonium quaternaires peuvent également être dérivés d'hétérocycles comportant 1, 2 ou 3 atomes d'azote et/ou de phosphore. Des radicaux tels que R⁶ peuvent également unir deux molécules telles que ci-dessus, comme par exemple R¹ R² N+ = CR³ - R⁶ -CR³ = N⁺ R¹ R² (X⁻)₂; R⁶ pouvant être un reste alkylène ou encore phénylène. A titre d'exemples on peut citer le chlorure de tétrabutylphosphonium, le chlorure de N-butylpyridinium, le bromure d'éthyl pyridinium, le chlorure de butyl-3 méthyl- 1 imidazolium, le chlorure de diéthylpyrazolium, le chlorhydrate de pyridinium, le chlorure de triméthylphényl ammonium .

Les dérivés organiques de l'aluminium selon l'invention ont pour formule générale AlR_{X}X₃₋ₓ dans laquelle R est un radical alkyl, linéaire ou ramifié, comportant 2 à 8 atomes de carbone, X étant le chlore ou le brome et x ayant une valeur égale à 1, 2 ou 3. A titre d'exemples on peut utiliser le dichloroéthylaluminium, le sesquichlorure d'éthylaluminium, le sesquichlorure d'isobutylaluminium, le dichloroisobutylaluminium et le chlorodiéthylaluminium.

Les composants des "sels fondus" tels que definis ci-dessus sont mis en oeuvre dans des rapports molaires A:B compris entre 1:0,5 et 1:3, de préférence entre 1: 0.8 et 1:2; B:C compris entre 1:0,01 et 1: 1,5 de préférence entre 1:0,01 et 1:1. Il est néanmoins nécessaire que les composants et leurs proportions soient tels que le mélange soit liquide à la température à laquelle se fait l'introduction du composé du tungstène ou du molybdène, bien que la réaction catalytique de disproportion puisse se faire à une température inférieure ou supérieure à la température de fusion de la composition catalytique.

Les composés entrant dans la composition selon l'invention, peuvent être mélangés dans un ordre quelconque. Le mélange peut se faire par une simple mise en contact suivie d'une agitation jusqu'à formation d'un liquide homogène. Ce mélange peut être fait en dehors du réacteur de disproportion ou de préférence dans ce réacteur.

Les composés du tungstène selon l'invention ont pour formule générale WX₆₋ₓ(OAr)ₓ et les composés du molybdène selon l'invention ont pour formule générale MoX ₅₋ₓ (OAᵣ)ₓ dans laquelle X représente le chlore ou le brome et Ar un groupement aromatique comportant au moins deux substituants hydrocarbonés en positions ortho, ortho', et x étant égal à 1 ou 2 . Le groupement aromatique est de préférence un groupement phényl, et les substituants des groupes phényl, alkyl, cycloalkyl, par exemple méthyl, isopropyl, tertiobutyl, cyclohexyl.

A titre d'exemples de composés du tungstène ou du molybdène utilisables selon l'invention on peut citer les complexes WCl₄ [O-2,6-C₆H₃(C₆H₅)₂]₂, WCl₄[O-2,4,6-C₆H₂(C₆H₅)₃], WCl₅[O-2,6-C₆H₃(C₆H₅)₂], WCl₄[O-2,6-C₆H₃(C₄H₉)₂]₂, MₒCl₃[O-2,6-C₆H₃(C₆H₅)₂]₂.

La concentration du composé du tungstène ou du molybdène dans le "sel fondu" est avantageusement comprise entre 5 et 200 mmol par litre, et de préférence entre 10 et 100 mmol par litre.

Les oléfines susceptibles d'être transformées par les compositions catalytiques selon l'invention sont le propylène, les n-butènes et les n-pentènes, seules ou en mélange, pures ou diluées par un alcane, telles qu'on les trouve dans des "coupes" issues des procédés de raffinage du pétrole, comme le craquage catalytique ou le craquage à la vapeur.

La réaction catalytique de disproportion des oléfines peut être conduite en système fermé, en système semi-ouvert ou en continu avec un ou plusieurs étages de réaction. Une vigoureuse agitation doit assurer un bon contact entre le ou les réactifs et la composition catalytique. La température de réaction peut être comprise entre -40 et +70 °C, de préférence entre -20 et +50 °C. On peut opérer au dessus ou en dessous de la température de fusion de la composition catalytique, l'état solide dispersé n'étant pas une limitation au bon déroulement de la réaction. La pression peut être comprise entre la pression atmosphérique et 20 MPa, de préférence entre la pression atmosphérique et 5 MPa. Les produits de la réaction sont séparés du système catalytique par simple décantation puis fractionnés.

Les exemples suivants illustrent l'invention sans en limiter la portée:

### EXEMPLE 1

### Préparation du solvant ionique.

On a mélangé à température ambiante 17,5 g (0,1 mole) de chlorure de butylméthyl imidazolium avec 12,8 g (0,096 mole) de chlorure d'aluminium sublimé (fraction molaire en chlorure d'aluminium: 0,489).

### Disproportion du pentène-2.

Un réacteur en verre de 100 ml muni d'un barreau aimanté pour assurer une bonne agitation et d'une double enveloppe permettant la circulation d'un liquide de régulation de température, a été purgé d'air et d'humidité, et maintenu sous atmosphère d'argon. A 30°C on y a introduit 83 mg (0,1 mmole) du complexe WCl₄[O-2,6-C₆H₃(C₆H₅)₂]₂ et injecté à l'aide d'une seringue 4,4g de la composition liquide ci-dessus préparée, 8 mL d'heptane contenant 0,245g (1,9 mmole) de dichloroéthylaluminium puis 10 mL de pentène-2.. On a mis l'agitation en route et après 4 heures on a laissé se décanter le "sel fondu" et on a soutiré la plus grande partie de la phase hydrocarbonée. On a recommencé l'opération deux fois. A ce moment on avait introduit au total 20 mL d'oléfines. L'analyse à montré que le mélange contenait 25% molaire de butène-2, 25% molaire d'hexène-3 et 50% molaire de pentène-2, c'est-à-dire était à l'équilibre thermodynamique de disproportion. L'opération a pu être recommencée plusieurs fois.

### EXEMPLE 2

### Préparation du solvant ionique.

On a mélangé à température ambiante 17,5 g (0,1 mole) de chlorure de butylméthyl imidazolium avec 16,02 g (0,12 mole) de chlorure d'aluminium sublimé (fraction molaire en chlorure d'aluminium: 0,545).

### Disproportion du pentène-2

On a opéré comme dans l'exemple 1 à ceci près qu'on a introduit 107 mg du complexe WCl_{4[}O-2,6-C₆H₃(C₆H₅)₂]₂, 4g du sel fondu préparé à cet effet et 0.4g (3,2 mmole) de dichloroéthylaluminium. L'analyse a montré que 2 charges successives de pentène-2 ont été conduites à l'équilibre thermodynamique.

### EXEMPLE 3

### Disproportion du pentène-2

On a opéré comme dans l'exemple 2 à ceci près qu'on a introduit 122 mg (0,13 mmole) du complexe WCl₄[O-2,4,6-C₆H₂(C₆H₅)₃] et 0,4 g (3,2 mmole) de dichloroéthylaluminium. L'analyse a montré que deux charges successives de pentène-2 ont été conduites à l'équilibre thermodynamique.

### EXEMPLE 4

### Disproportion du pentène-2

On a opéré comme dans l'exemple 1 à ceci près qu'on a introduit 72 mg (0,07 mmole) du complexe WCl₄[O-2,4,6-C₆H₂(C₆H₅)₃] et 0,49 g (3,9 mmole) de dichloroéthylaluminium. Le résultat a été identique à celui de l'exemple 1.

### EXEMPLE 5

### Préparation du solvant ionique

On a mélangé à température ambiante 17,5 g (0,1 mole) de chlorure de butylméthyl imidazolium avec 11,9 g (0,086 mole) de chlorure d'aluminium sublimé (fraction molaire en chlorure d'aluminium: 0,47).

### Disproportion du pentène-2

On a opéré comme dans l'exemple 1 à ceci près qu'on a introduit 62 mg (0,1 mmole) du complexe WCl₅[O-2,6-C₆H₃(C₆H₅)₂], 3,25g du sel fondu préparé à cet effet et 0,245g (1,9 mmole) de dichloroéthylaluminium. Au bout de 4 heures la conversion était de 33%.

### EXEMPLE 6

### Disproportion du pentène-2

On a opéré comme dans l'exemple 1 à ceci près qu'on a introduit 100 mg (0,14 mmole) du complexe WCl₄[O-2,6-C₆H₃(C₄H₉)₂]₂ et 0,49g (3,9 mmole) de dichloroéthylaluminium. Après 4 heures de réaction la conversion était de 40%.

### EXEMPLE 7

### Disproportion du pentène-2

On a opéré comme dans l'exemple 6 à ceci près qu'on a introduit 100 mg (0,14 mmole) du complexe MₒCl₃[O-2,6-C₆H₃(C₆H₅)₂]₂ et 0,49g (3,9 mmole) de dichloroéthylaluminium. Après 4 heures de réaction la conversion était de 40%.

## Revendications

1. Composition catalytique comportant au moins un composé du tungstène ou du molybdène, le composé du tungstène ayant une formule WX₆₋ₓ(OAr)ₓ et le composé du molybdène ayant une formule MoX₆₋ₓ(OAr)ₓ dans laquelle X représente le chlore ou le brome et Ar un groupement aromatique comportant au moins deux substituants hydrocarbonés en positions ortho, ortho', et X étant égal à un ou deux, dissous au moins en partie dans un milieu non-aqueux à caractère ionique résultant de la mise en contact d'au moins un halogénure d'aluminium (A), avec au moins un halogénure d'ammonium quaternaire ou/et d'au moins un halogénure de phosphonium quaternaire (B), et avec au moins un composé organométallique de l'aluminium.(C).

2. Composition catalytique selon la revendication 1 dans laquelle l'halogénure d'ammonium quaternaire est choisi dans le groupe constitué par le chlorure de N-butylpyridinium, le bromure d'éthylpyridinium, le chlorure de butyl-3-méthyl-1 imidazolium, le chlorure de diéthylpyrazolium, le chlorhydrate de pyridinium et le chlorure de triméthylphényl ammonium.

3. Composition catalytique selon l'une des revendications précédentes dans laquelle l'halogénure de phosphonium quaternaire est le chlorure de tétrabutylphosphonium.

4. Composition catalytique selon l'une des revendications précédentes dans laquelle l'halogénure d'aluminium est choisi dans le groupe formé par le chlorure d'aluminium et le bromure d'aluminium.

5. Composition catalytique selon l'une des revendications précédentes dans laquelle le milieu non-aqueux contient également un dérivé organique de l'aluminium (C) de formule générale AlRₓX₃₋ₓ où R est un radical alkyl, linéaire ou ramifié, comportant de 2 à 8 atomes de carbone, X est le chlore ou le brome et x est égal à 1, 2 ou 3.

6. Composition catalytique selon la revendication 5 dans laquelle le dérivé organique de l'aluminium est choisi dans le groupe formé par le dichloroéthylaluminium, le dichloroisobutylaluminium, le chlorodiéthylaluminium, le sesquichlorure d'éthylaluminium, le sesquichlorure d'isobutylaluminium.

7. Composition catalytique selon l'une des revendications précédentes, dans laquelle le rapport molaire A : B est compris entre l:0,5 et 1:3, le rapport molaire B : C est compris entre 1:0,01 et 1:1,5

8. Composition catalytique selon l'une des revendications précédentes dans laquelle le rapport molaire A : B est préférentiellement compris entre 1:0,8 et 1:2, le rapport molaire B : C est préférentiellement compris entre 1:0,1 et 1:1.

9. Composition catalytique selon l'une des revendications précédentes, dans laquelle le composé du tungstène a pour formule générale WX₆₋ₓ(OAr)ₓ dans laquelle X représente le chlore ou le brome et Ar un groupement aromatique comportant au moins deux substituants hydrocarbonés en positions ortho, ortho', et x étant égal à 1 ou 2 .

10. Composition catalytique selon l'une des revendications précédentes, dans laquelle le composé du molybdène a pour formule générale MoX₅₋ₓ(OAr)ₓ dans laquelle X représente le chlore ou le brome et Ar un groupement aromatique comportant au moins deux substituants hydrocarbonés en positions ortho, ortho', et x étant égal à 1 ou 2 .

11. Procédé pour la disproportion ou la codisproportion d'au moins une oléfine, caractérisé en ce que l'oléfine est mise en contact d'au moins un composé du tungstène ou du molybdène , ledit composé étant dissous au moins en partie, dans un milieu non-aqueux à caractère ionique, ledit milieu résultant de la mise en contact d'au moins un halogénure d'aluminium avec au moins un halogénure d'ammonium quaternaire et/ou au moins un halogénure de phosphonium quaternaire et avec au moins un composé organométallique de l'aluminium.

12. Procédé selon la revendication 11 dans lequel la température de réaction est comprise entre -40°C et +70°C et la pression comprise entre 0,1 et 20 MPa.

13. Procédé selon l'une des revendications précédentes dans lequel la température de réaction avec l'oléfine est comprise entre -20°C et +30°C.

## Claims

1. A catalytic composition comprising at least one tungsten or molybdenum compound, the tungsten compound has formula WX_{6-X}(OAr)ₓ and the molybdenum compound has formula MoX₅₋ₓ(OAr)ₓ wherein X represents chlorine or bromine and Ar an aromatic group containing at least 2 hydrocarbon substituents in the ortho, ortho's positions and x equals 1 or 2, which is at least partially dissolved in a non aqueous ionic medium produced by bringing at least one aluminium halide (A) into contact with at least one quaternary ammonium halide and/or at least one quaternary phosphonium halide (B) and with at least one organometallic aluminium compound (C).

2. A catalytic composition according to claim 1, wherein the quaternary ammonium halide is selected from the group constituted by N-butylpyridinium chloride, ethylpyridinium bromide, 3-butyl-1-methyl imidazolium chloride, diethylpyrazolium chloride, pyridinium hydrochloride and trimethylphenyl ammonium chloride.

3. A catalytic composition according to claim 1 or claim 2, wherein the quaternary phosphonium halide is tetrabutylphosphonium chloride.

4. A catalytic composition according to any one of the preceding claims, wherein the aluminium halide is selected from the group formed by aluminium chloride and aluminium bromide.

5. A catalytic composition according to any one of the preceding claims wherein the non aqueous medium also contains an organic aluminium derivative (C) with general formula AlRₓX₃₋ₓ where R is a linear or branched alkyl radical containing 2 to 8 carbon atoms, X is chlorine or bromine and x equals 1, 2 or 3.

6. A catalytic composition according to claim 5, wherein the organic aluminium derivative is selected from the group formed by dichloroethylaluminium, dichloroisobutylaluminium, chlorodiethylaluminium, ethylaluminium sesquichloride and isobutylaluminium sesquichloride.

7. A catalytic composition according to any one of the preceding claims, wherein the molar ratio A:B is between 1:0.5 and 1:3, and the molar ratio B:C is between 1:0.01 and 1:1.5.

8. A catalytic composition according to any one of the preceding claims, wherein the molar ratio A:B is preferably between 1:0.8 and 1:2 and the molar ratio B:C is preferably between 1:0.1 and 1:1.

9. A catalytic composition according to any one of the preceding claims, wherein the tungsten compound has general formula WX₆₋ₓ(OAr)ₓ, where x represents chlorine or bromine and Ar represents an aromatic group containing at least two hydrocarbon substituents in the ortho, ortho' positions, and x equals 1 or 2.

10. A catalytic composition according to any one of the preceding claims, wherein the molybdenum compound has general formula MoX₅₋ₓ(OAr)ₓ, where X represents chlorine or bromine and Ar represents an aromatic group containing at least two hydrocarbon substituents in the ortho, ortho' positions, and x equals 1 or 2.

11. A process for disproportion or codisproportion of at least one olefin, characterised in that the olefin is brought into contact with at least one tungsten or molybdenum compound, said compound being at least partially dissolved in an ionic non aqueous medium, said medium being produced by bringing at least one aluminium halide into contact with at least one quaternary ammonium halide and/or at least one quaternary phosphonium halide and with at least one organometallic aluminium compound.

12. A process according to claim 11, wherein the reaction temperature is between -40°C and +70°C, and the pressure is between 0.1 and 20 MPa.

13. A process according to any one of the preceding claims, wherein the reaction with the olefin is carried out at a temperature of between -20°C and +30°C.

## Patentansprüche

1. Katalytische Zusammensetzung, die enthält mindestens eine Wolframoder Molybdän-Verbindung der Formel WX₆₋ₓ(OAr)ₓ bzw. der Formel MoX₅₋ₓ(OAr)ₓ worin X für Chlor oder Brom, Ar für eine aromatische Gruppe, die mindestens zwei Kohlenwasserstoff-Substituenten in den ortho-, ortho'-Positionen trägt und x für die Zahl 1 oder 2 stehen, gelöst mindestens zum Teil in einem nicht-wäßrigen Medium mit ionischem Charakter, das resultiert aus dem Inkontaktbringen mindestens eines Aluminiumhalogenids (A) mit mindestens einem quaternären Ammoniumhalogenid und/oder mindestens einem quaternären Phosphoniumhalogenid (B) und mit mindestens einer metallorganischen Aluminiumverbindung (C).

2. Katalytische Zusammensetzung nach Anspruch 1, in der das quaternäre Ammoniumhalogenid ausgewählt wird aus der Gruppe, die besteht aus N-Butylpyridiniumchlorid, Ethylpyridiniumbromid, 3-Butyl-1-methyl-imidazoliumchlorid, Diethylpyrazoliumchlorid, Pyridiniumhydrochlorid und Trimethylphenylammoniumchlorid.

3. Katalytische Zusammensetzung nach einem der vorhergehenden Ansprüche, in der das quaternäre Phosphoniumhalogenid das Tetrabutylphosphoniumchlorid ist.

4. Katalytische Zusammensetzung nach einem der vorhergehenden Ansprüche, in der das Aluminiumhalogenid ausgewählt wird aus der Gruppe, die besteht aus Aluminiumchlorid und Aluminiumbromid.

5. Katalytische Zusammensetzung nach einem der vorhergehenden Ansprüche, in der das nicht-wäßrige Medium außerdem ein organisches Aluminium-Derivat (C) der allgemeinen Formel AlRₓX₃₋ₓ enthält, worin R für einen linearen oder verzweigten Alkylrest mit 2 bis 8 Kohlenstoffatomen, X für Chlor oder Brom und x für die Zahl 1, 2 oder 3 stehen.

6. Katalytische Zusammensetzung nach Anspruch 5, in der das organische Aluminium-Derivat ausgewählt wird aus der Gruppe, die besteht aus Dichlorethylaluminium, Dichlorisobutylaluminium, Chlordiethylaluminium, Ethylaluminiumsesquichlorid und Isobutylaluminiumsesquichlorid.

7. Katalytische Zusammensetzung nach einem der vorhergehenden Ansprüche, in der das Molverhältnis A:B zwischen 1:0,5 und 1:3 und das Molverhältnis B:C zwischen 1:0,01 und 1:1,5 liegen.

8. Katalytische Zusammensetzung nach einem der vorhergehenden Ansprüche, in der das Molverhältnis AB vorzugsweise zwischen 1:0,8 und 1:2 und das Molverhältnis B:C vorzugsweise zwischen 1:0,1 und 1:1 liegen.

9. Katalytische Zusammensetzung nach einem der vorhergehenden Ansprüche, in der die Wolfram-Verbindung die allgemeine Formel hat WX₆₋ₓ(OAr)ₓ, in der X für Chlor oder Brom, Ar für eine aromatische Gruppe, die mindestens zwei Kohlenwasserstoff-Substituenten in den ortho-, ortho'-Positionen trägt, und x für die Zahl 1 oder 2 stehen.

10. Katalytische Zusammensetzung nach einem der vorhergehenden Ansprüche, in der die Molybdän-Verbindung die allgemeinen Formel hat MoX₅₋ₓ(OAr)ₓ, in der X für Chlor oder Brom, Ar für eine aromatische Gruppe, die mindestens zwei Kohlenwasserstoff-Substituenten in den ortho-, ortho'-Positionen trägt, und x für die Zahl 1 oder 2 stehen.

11. Verfahren zur Disproportionierung oder Co-Disproportionierung mindestens eines Olefins, dadurch gekennzeichnet, daß das Olefin mit mindestens einer Wolfram- oder Molybdän-Verbindung, die mindestens zum Teil in einem nicht-wäßrigen Medium mit ionischem Charakter gelöst ist, in Kontakt gebracht wird, wobei das genannte Medium resultiert aus dem Inkontaktbringen mindestens eines Aluminiumhalogenids mit mindestens einem quaternären Ammoniumhalogenid und/oder mindestens einem quaternären Phosphoniumhalogenid und mit mindestens einer metallorganischen Aluminiumverbindung.

12. Verfahren nach Anspruch 11, bei dem die Reaktionstemperatur zwischen -40 und +70°C und der Druck zwischen 0,1 und 20 MPa liegen.

13. Verfahren nach einem der vorhergehenden Ansprüche, bei dem die Temperatur der Reaktion mit dem Olefin zwischen -20 und +30°C liegt.
